# EUROPEAN PATENT SPECIFICATION

(11) **EP 2 283 824 B1**
(45) Date of publication and mention of the grant of the patent: **19.04.2017**
(21) Application number: 09166824.4
(22) Date of filing: 30.07.2009
(51) Int. Cl.: A61K 31/7048, A61K 9/22, A61K 31/138

(54) **Compositions and formulations based on swellable matrices for sustained release of poorly soluble drugs such as clarithromycin**
Zusammensetzungen und Formulierungen, die auf quellbaren Matrizen basieren, zur verzögerten Freisetzung von schlecht löslichen Arzneimitteln, wie zum Beispiel Clarithromycin
Compositions et formules basées sur des matrices gonflables pour la libération prolongée de médicaments, comme clarithromycin, faiblement solubles

(43) Date of publication of application: 16.02.2011
(73) Proprietor: Special Product's Line S.p.A., 00040 Pomezia (IT); So. Se. Pharm S.r.l., 00040 Pomezia (IT)
(72) Inventor: Camponeschi, Claudio, 00040, Pomezia (IT); Colombo, Paolo, 43100, Parma (IT)
(74) Representative: Palladino, Saverio Massimo

(56) References cited:
- WO-A-2004/006888
- WO-A-2005/023221
- US-A1- 2005 053 657
- US-A1- 2008 050 430
- ISHIKAWA TATSUYA ET AL: "Effect of hydroxypropylmethylcellulose (HPMC) on the release profiles and bioavailability of a poorly water-soluble drug from tablets prepared using macrogol and HPMC" INTERNATIONAL JOURNAL OF PHARMACEUTICS (KIDLINGTON), vol. 202, no. 1-2, 20 July 2000 (2000-07-20), pages 173-178, XP002561511 ISSN: 0378-5173

## Description

### FIELD OF THE INVENTION

The present invention concerns the discovery of new compositions and formulations for oral drug modified release products, capable to provide a sustained release of poorly soluble drugs whose solubility depends on the pH.

### BACKGROUND OF THE INVENTION

New drug dosage forms facilitating the human therapeutic practice are needed for making efficient the treatment of several diseases. Typical dosage forms that improve the efficiency of the drug administration are the modified release preparations. These products allow the reduction of the daily number of dosage administration, the improvement of the blood level of drug and the increase of the compliance of the patient. Classical modified release dosage forms are tablet made of a mixture of hydrophilic polymer and drug. These preparations are defined swellable matrices from which the drug is released through the diffusive layer constituted by the gel produced on the matrix surface by polymer swelling. The release mechanism is by diffusion when the drug is soluble or by matrix erosion when the drug is insoluble. In general, it is not difficult to make modified release matrices in case of soluble drugs, whereas the control of release of insoluble (or poorly soluble) drugs is more difficult. Though insoluble or "poorly" soluble (sometimes are also used the terms "scarcely" or "sparingly" soluble) are relative terms, these are well recognized and accepted in the field of drugs, and in general are referred to compounds having a solubility in water of less than about 10 mg/ml (see, e.g., US Pat. No. 5,145,684).

Besides, many interesting insoluble or poorly soluble drugs have a solubility profile decreasing in conditions of pH increasing from 1.0 to 7.0; among these, may be cited antibiotics, such as neomycin, erythromycin, clarithromycin, flurithromycin or roxithromicin; antivirals, such as acyclovir; chemotherapeutics, such as ciprofloxacin; and antihipertensives, as nifedipine or nicardipine; as an example, approximate solubility values of clarithromycin and flurithromycin in water at different pH values are as follows:

| pH | Solubility (mg/ml) | |
|---|---|---|
| | clarithromycin | flurithromycin |
| 1.0 | 26.1 | 22.0 |
| 2.0 | / | 11.8 |
| 4.5 | 3.9 | 4.2 |
| 6.0 | 3.3 | 0.3 |

All the drugs having the above mentioned characteristics, namely, a poor solubility that decreases with increasing pH, could benefit of a modified release preparation; among these, widely studied are those of the erythromicyn (macrolide) class of antibiotics. Two antibiotics belonging to this class usually administered orally are clarithromycin of flurithromycin ethylsuccinate, so the following discussion will be referred mostly to these two drugs, but the teachings herein are valid also for other poorly soluble drugs whose solubility depends on pH, in particular of the macrolide family.

Patent literature reports a number of formulations in which clarithromycin is associated with other components in order to improve its solubility and control its release.

Hydrophilic polymers for the production of matrices for the administration of clarithromycin are described in various patents. Of these, the most commonly cited one is hydroxypropylmethylcellulose (HPMC) at various molecular weights. Several patent documents relate the use of high viscosity HPMC (see, e.g., US 2004/0247679 A1) or low viscosity HPMC (see, e.g., WO 98/46239 A1, WO 03/099197 A2, WO 2005/023221 A, WO 2004/006888 A, US 2008/050430 A1, US 2005/053657 A, WO 2004/100880 A2), in concentrations up to 50%.

Patent application WO 03/017981 A1 discloses the use of high or low viscosity HPMC in low concentrations, between 0.1 and 4.5% by weight of the overall dosage form.

WO 2004/066910 A2 teaches the use of a mixture of polyethylenoxides (PEO) of various molecular weights for the modified release of antibiotics; in this case derivatives of starch are also used as auxiliary substances, acting synergistically to prolong the release of the active substance.

To increase the water solubility of clarithromycin, patent application WO 97/22335 A1 teaches the addition of organic acids to pharmaceutical compositions; documents WO 98/46239 A1, EP 1302205 A1 and WO 01/49246 A2 disclose in particular the use of citric acid in combination with the drug in the dosage form.

Other documents propose different systems for modifying drug release properties of dosage forms of clarithromycin. For instance, patent applications WO 01/10405 A1 and WO 03/097018 A1 teach formulations containing clarithromycin in which gas forming agents are used to obtain a low density matrix with floating properties, due to the entrapment of gas bubbles in the gel layer; WO 2004/112711 A2 describes the possibility of obtaining modified release solid dosage forms from the mixture of two separately produced granulates, each one containing clarithromycin and HPMC in different proportions; finally, WO 00/48607 A1 teaches modified release formulations obtained using mixtures of triglycerides of highly saturated fatty acids and HPMC.

A problem is still open in the field of modified release formulations for oral delivery of drugs having a solubility that changes with pH (such as macrolide derivatives) is that presently available formulations generally show a profile of release of the drug that decreases with increasing pH; in other words, despite the formulation with additives added to enhance drug release at nearly neutral pH values, the known formulations still show a decrease in the rate of release of the drug when pH raises from about 1.0 to values in the range of about 6.0 to 7.0. As a consequence, the dissolution rate of these drugs changes in the different environments of the GI tract (passing from a strong acid environment in the stomach, to the nearly neutral ambient in the intestinal tract); this leads to changes in drug release rates of formulations containing these drugs. In general, the release mechanism from swellable matrices for drugs having low solubility is the erosive one. However, the erosive release mechanism is quite difficult to create and control and shows high variability. Thus, new formulation of modified release preparations of insoluble drugs having solubility that changes with pH requires innovative approaches.

It is thus an object of the invention to provide a sustained release composition for poorly soluble drugs having a pH-dependent solubility, in particular of the macrolide family, showing sustained release of the drug regardless the pH.

Other objects of the invention are to provide an oral dosage form of drugs prepared using said composition, and methods for producing said dosage form.

### SUMMARY OF THE INVENTION

According to its first aspect, the invention provides a sustained release composition for poorly soluble drugs having a pH-dependent solubility, in the form of a swellable matrix comprising:
- from 10 to 30% by weight of a rate controlling hydrophilic swellable polymer being hydroxypropylmethylcellulose having a viscosity value in the range from 4,000 to 15,000 mPa.s., or a blend of two hydroxypropylmethylcellulose fractions of different viscosity, both in the range from 50 to 15,000 mPa.s, the mean viscosity of the blend being comprised between 1,000 and 3,000 mPa.s;
- from 0.5 to 5% by weight of a disgregation promoter selected among polyethylenglycol of molecular weight between 200 and 8,000 Da; polyethylenoxide of molecular weight lower than about 10,000 Da; polyethylenoxide derivatives obtained by terminating polyethylenoxide chains with either alkyesters with a hydrophilic-lipophilic balance value higher than 15 or sorbitan fatty esters with a hydrophilic-lipophilic balance value higher than 14.9; poloxamers; and polyvinyl alcohol of molecular weight comprised between 40,000 and 100,000 Da;
- from 1 to 5% by weight of polyvinylpyrrolidone having average molecular weight of 50,000 Da; and
- from 40 to 85% by weight of at least one such drug.

In the rest of the description, by the term "sustained release" will be meant a release that does not show the rate decrease when passing from strong acidic to nearly neutral pH, as is the case for presently known formulations; and by "poorly soluble drugs having a pH-dependent solubility" will be meant a drug having a solubility in the range of a few tens of mg/ml in strongly acid environments, which decreases with increasing pH.

In particular, the compositions of the invention preferably comprise a macrolide drug, hydroxypropylmethylcellulose (HPMC) as the rate controlling hydrophilic swellable polymer, polyethylenglycol (PEG) as the disgregation promoter and polyvinylpyrrolidone (PVP) as the binder. In the description that follows, the rate controlling hydrophilic swellable polymer will also be referred to simply as "rate controlling polymer".

Compositions similar to those of the present invention, but without a disgregation promoter, are known in the field. In general, the rate-controlling polymer swells in contact with water and forms a gel layer around the dosage forms. At low pH values, e.g., of about 1.0 and up to about 4.5, the drugs, having an appreciable solubility and dissolution rate, contribute to create a thick outer gel layer on the external surface of the dosage form; at these pH values, thus, the drugs are released through a diffusive delivery mechanism through the gel layer. The extraction of the drugs, in turn, favors the water intake, so that the gel layer grows with time inwardly in the dosage form reaching a thickness providing a sufficient resistance for keeping the matrix coherent. At higher pH values, e.g., higher than about 4.5, the drugs, becoming essentially non soluble, remain in solid state and are not able to cross the gel layer anymore; in this case, drug release requires the disgregation of the gel layer, and the mechanism of drug delivery becomes erosive. The inventors have now discovered that the disgregation promoter component affects the consistency of the gel layer by weakening it, likely due to competition for water inside the gel structure; this has essentially no effect on the diffusive delivery mechanism occurring at low pH values, while it increases the disgregation rate of the matrix at higher pH values, thus accelerating the drug release that happens through the erosive mechanism. The net result is that the two different delivery mechanisms, active in different pH ranges, combine to yield a sustained rate of release of the drug along the whole GI tract.

### BRIEF DESCRIPTION OF THE DRAWINGS

Further features and advantages of the present invention will be more apparent from the following detailed description by way of non-limitative example and illustrated in the accompanying drawings, in which:
- Figure 1 is a graph showing the *in-vitro* dissolution profiles at variable pH of clarithromycin matrix tablets of the invention, of a known clarithromycin-based drug, and of tablets produced not according to the invention;
- Figure 2 is a graph showing the *in-vitro* dissolution profiles at variable pH of flurithromycin matrix tablets having two different formulations according to the invention.

### DETAILED DESCRIPTION OF THE INVENTION

The compositions of the invention comprise a rate controlling polymer in total amounts in the range from about 10 to 30% of the overall weight of the composition; a disgregation promoter in amounts in the range from about 0.5 to 5% of the weight of the composition; a binder in amounts in the range from about 1 to 5% of the weight of the composition; and at least one poorly soluble drug, having a pH-dependent solubility, in total amounts in the range from about 40 to 85% of the weight of the composition.

The drugs useful for preparing the compositions of the invention are all the drugs that are poorly soluble and show a pH-dependent solubility as discussed above, such as the drugs of the macrolide family; in particular, the compositions of the invention are preferably manufactured with the drugs neomycin, erythromycin, clarithromycin, flurithromycin, roxithromicin, acyclovir, ciprofloxacin, nifedipine or nicardipine.

The rate controlling polymer is a pharmaceutically acceptable hydrosoluble and swellable polymer, preferably hydroxypropylmethylcellulose (HPMC) having a viscosity value in the range from about 4,000 to 15,000 milliPascal · second (mPa·s). Preferably, this component is used in the form of a blend of two HPMC fractions of different viscosity, both in the range from 50 to 15,000 mPa·s; in this case, the mean viscosity of the blend is comprised between about 1,000 and 3,000; the mean viscosity can either be determined by trial tests, or it can be preset with the aid of blending charts provided in technical bulletins of commercially sold HPMCs; for instance, particularly preferred for use in the compositions of the invention are the HPMCs of the series Metolose 90SH, sold by Shin-Etsu Chemical Co., Ltd., for which such a blending chart is provided by the manufacturer.

The disgregation promoter is in its turn a hydrophilic polymer, chosen among: polyethylenglycol (PEG) of molecular weight (MW) between 200 and 8,000 dalton (Da); polyethylenoxide (PEO) of MW lower than about 10,000 Da; PEO derivatives obtained by terminating PEO chains with either alkyesters with a hydrophilic-lipophilic balance (HLB) value higher than 15 or sorbitan fatty esters with a HLB value higher than 14.9; poloxamers, namely, a family of triblock polyethylenoxide-polypropylenoxide-polyethylenoxide (PEO-PPO-PEO) copolymers, sold commercially with the name Pluronic^{®} (a registered mark of BASF Corp.); and polyvinyl alcohol (PVA) of MW comprised between 40,000 and 100,000 Da. Preferred for the use as disgregation promoter in the compositions of the present invention is PEG, in particular solid fractions of PEG, having MW generally comprised between 1,500 and 8,000 Da, and more particularly PEG of MW of about 6,000 Da (PEG 6000); useful PEG forms are commercially available, e.g., as Carbowax^{®} from Union Carbide Chemicals & Plastics Technology Corp.

Finally, the binder is preferably polyvinylpyrrolidone (PVP), preferably in the form marketed by several manufacturers with the name PVP K30, having average MW of about 50,000 Da.

The compositions of the invention may also contain other components with different functions, as known in the field; for instance, these compositions can comprise citric acid, acting as promoter of macrolides solubility; mannitol, acting as promoter of wettability and of water intake; talc and magnesium stearate as lubricants; sodium dodecyl sulfate (SDS), as wetting agent; and sodium starch glycolate, with the function of disintegrating agent; given the intended function of this latter excipient, when this is used, hydrophilic excipients such as citric acid and mannitol are to be avoided. When present, these further excipients and additives are added in amounts up to 10-15% by weight (b.w.) of the composition in case of citric acid and mannitol; up to 2-3% b.w. in case of talc; up to about 1% in case of magnesium stearate; up to about 4% b.w. in case of sodium starch glycolate; and up to 0.5% b.w. in case of SDS.

In its second aspect, the invention is about the sustained release dosage forms that can be produced by using the compositions described so far.

The compositions of the invention may be manufactured in two different ways; in a first embodiment, all components of the compositions are simply mixed with known techniques in the desired weight ratios, kneaded, sieved, dried to remove possible solvents, lubricated and compressed in a suitable tableting machine; in this case, dosage forms are obtained of thoroughly homogeneous composition.

Alternatively, it is possible to knead all desired components but the rate controlling polymer, producing a granulate with said mixture, and mixing the thus obtained granules with the rate controlling polymer (this latter preferably in the form of a blend of two fractions of different MW of the same polymer), so that the drug-containing granules are dispersed in a matrix of pure rate controlling polymer, commonly HPMC. Said granules have preferably dimensions lower than about 800 µm. This second embodiment of dosage forms is slightly more complex to produce, but offers advantages in the linearity of the release rate and in the handling of the starting materials, as discussed below with reference to the methods for producing the dosage forms of the invention.

As known in the field, the dosage forms obtained with the compositions of the invention are preferably coated with a film, that makes the dosage form more slippery (thus favoring its swallowing) and masks the composition taste during the transit of the dosage form in the mouth. These coatings are well known in the field and need not be described in detail here; a preferred coating for the compositions of the invention comprises, by weight, about 46.7% of hypromellose (a form of HPMC), 26.7% of propylene glycol, 2% of sorbitan mono-oleate, 1.7% vanillin, 2% of hydroxypropyl cellulose, 1.7% of sorbic acid and 19.3% of titanium dioxide. The coating has generally a thickness comprised between about 100 and 500 microns.

The two dosage forms described above, although produced with different procedure, are prepared starting with the same percentages of components, simply with different structural arrangements; that is, there is no need to provide for an adjusted amount of one or more components for making dosage forms made up of drug-containing granules in a matrix of pure rate controlling polymer, compared to the case of thoroughly homogeneous dosage forms. Typically, these dosage forms are in the form of tablets of weight comprised between about 0.6 and 1.2 g, more than 50% of which is represented by the drug; these tablets may have various shapes (circular, square, rectangular, lozenges, ...), with the higher dimension (typically the length) generally lower than about 2 cm.

In its last aspect, the invention concerns methods for producing the sustained release dosage forms described above.

Dosage forms of the first embodiment described above are produced by first mixing the desired drug with the rate controlling polymer, this latter preferably in two fractions of different average MW; mixing may take place for instance by means of a sigma mixer, and can be continued for times between about 5 minutes and one hour; this first mixture is then added to the disgregation promoter and the binder, these latter preferably in form of a solution, e.g. a hydroalcoholic solution; excipients such as citric acid or mannitol may be added in this phase to the solution. The wet mixture is then preferably granulated, in order to have a more handleable form; this step can be accomplished for instance by forcing the wet mixture through a mesh having openings of desired dimensions, for instance between 1 and 2 mm. The thus obtained granules are then dried in a fluid bed, for instance at air inlet temperature between room temperature and 50 °C. Finally, lubricant additives such as talc and magnesium stearate may be added to the dried granules, e.g. again by means of a tumbling mixer, for times generally lower than half a hour. This latter mixture is then tableted by means of a tablet machine to produce the desired dosage forms, for instance by means of an alternative tableting machine equipped with punches having the desired shape.

Dosage forms according to the second embodiment are produced by the following procedure. First, it is prepared a granulate of the desired drug with soluble adjuncts such as citric acid and mannitol (if these are foreseen in the composition); the pure drug, or the first mixture prepared above, are kneaded with a solution containing the disgregation promoter and the binder; the wet mixture produced in the previous step is granulated by forcing it through a mesh, in this case generally of lower dimensions compared to the first embodiment (e.g., 0.8 mm), and the granules are dried; the dried granulate is then mixed with the rate controlling polymer (the latter preferably in two fractions of different MW); other additives, such as lubricants, are added; and finally, the mixture is subdivided in portions of essentially equal weight by a dosing machine and compressed in a tableting machine. Equipments and operating conditions of this second method, when not specified, are the same as those described for similar steps of the first method.

This second method provides a more linear release rate and offers the advantage of a better handleability of powders and granules; in fact, most of the drugs suitable for the invention have critical rheological characteristics, being not well flowing in automated transport means or ducts, and giving rise, in the tableting step, to problems due to sticking to the punches and moulds walls. The incorporation of the drugs in granules with disgregation promoter and binder, and the subsequent mixing of such granules with the rate controlling polymer, avoid these problems.

The sustained release dosage forms produced as described above are preferably then coated with the previously described overlayer with methods known in the field.

The invention will be further illustrated by means of the following examples.

### EXAMPLE 1

This example describes the manufacture of sustained release dosage forms containing clarithromycin in granules dispersed in a HPMC matrix.

1000 tablets of a dosage form according to the invention are produced by using the compounds listed in Table 1, in weigths reported in the same table. Clarithromycin, mannitol and citric acid are first mixed for 20 minutes in a tumbling mixer. Then, the mixture is kneaded with a hydroalcoholic solution containing PVP K30 (10%), PEG 6000 (5%) e SDS (0.2%). The wet mixture is forced through a mesh of 500 µm and dried in a fluid bed at air inlet temperature of 40 °C. Dried granulate is mixed for 20 minutes with two fractions of HPMC (Metolose), talc and magnesium stearate. The mixture is then compressed using an alternative tableting machine equipped with punches having an oblong shape (major axis 17 mm; minor axis 7.6 mm).

**Table 1**

| **Component** | **Weight (g)** |
|---|---|
| Clarithromycin | 500 |
| Mannitol | 100 |
| Citric acid | 100 |
| Metolose 90SH 100SR | 70 |
| Metolose 90SH 4000SR | 30 |
| PVP K30 | 22.5 |
| PEG 6000 | 11.25 |
| Sodium dodecyl sulphate | 0.45 |
| Talc | 8.44 |
| Mg stearate | 4.12 |

Taking into account the residual water content after granulation (3.84%), the weight of a tablet containing 500 mg of clarithromycin results to be about 881 mg; in particular, the weight of 10 tablets randomly sampled out of the 1000 tablets produced is equal to 880.75 ± 12.79 mg, the thickness is 6.18 ± 0.01 mm, and the chlaritromycin content is 99.99% of the theoretical value of 500 mg.

### EXAMPLE 2

This example describes the manufacture of dosage forms containing flurithromycin in a composition homogeneous throughout the dosage form.

Two series of 1000 tablets each of dosage forms according to the invention are prepared using the compounds and weights reported in Table 2; the two series differ for a different ratio of weights of the two forms of HPMC. Flurithromycin, Metolose 100SR and Metolose 4000SR are mixed for 20 minutes in a tumbling mixer. Then, the mixture is kneaded with a hydroalcoholic solution containing PVP K30 (10%), PEG 6000 (5%) and SDS (0.1%). The wet mixture is then forced through a mesh of 1500 µm and dried in a fluid bed at air inlet temperature of 40 °C. Dried granulate is mixed for 20 minutes with talc and magnesium stearate. The mixture is then dosed in 1000 portions of essentially equal weight, and these portions are formed into tablets by compression using an alternative tableting machine equipped with punches having an oblong shape (major axis 20 mm; minor axis 9 mm).

**Table 2**

| **Component** | **Weight (g)** | |
|---|---|---|
| | Formulation A | Formulation B |
| Flurithromycin Ethylsuccinate | 877.7 | 877.7 |
| Metolose 90SH 100SR | 56.15 | 37.43 |
| Metolose 90SH 4000SR | 37.43 | 56.15 |
| PVP K30 | 32 | 32 |
| PEG 6000 | 16 | 16 |
| Sodium dodecyl sulphate | 0.32 | 0.32 |
| Sodium starch glycolate | 43.1 | 43.1 |
| Talc | 32.3 | 32.3 |
| Mg stearate | 10.8 | 10.8 |

Taking into account the residual water content after the granulation (0.20%), tablet weight containing 750 mg of flurithromycin was 1109 mg.

Average hardness, average thickness and average flurithromycin content (as percentage of the theoretical value) are measured on 10 tablets randomly sampled for each of the two formulations; these values are reported in Table 3.

**Table 3**

| | Hardness (kg) | Thickness (mm) | Content (%) |
|---|---|---|---|
| Formulation A | 9.0 ± 0.1 | 7.47 ± 0.04 | 102 ± 8 |
| Formulation B | 7.0 ± 0.1 | 7.55 ± 0.01 | 95 ± 4 |

### EXAMPLE 3 (COMPARATIVE)

This example describes the manufacture of dosage forms containing clarithromycin but without a disgregation promoter.

The procedure of example 1 is repeated, without adding the amount of 11.25 g of PEG 6000 adopted in said example. The residual humidity of granulate was 3.34%, the weight of the prepared tablet was 885 ± 15 mg and the thickness 6.20 ± 0.02 mm.

### EXAMPLE 4

This example describes the results of an *in-vitro* test of release with time of clarithromycin-containing dosage forms of the invention.

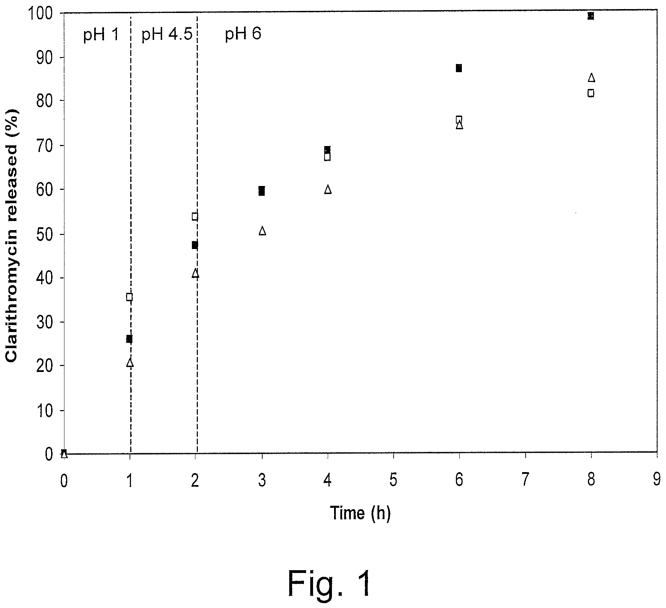

A tablet prepared as described in example 1 is placed in a vessel having a metallic net at the bottom (to avoid sticking of the tablet to the bottom wall of the vessel) and containing a first solution of HCl at pH = 1.0; the solution is kept under stirring for one hour by means of a rotating paddle. At the end of this period, the tablet is quickly removed from the vessel by means of a teflon-coated tweezers and immediately immersed in a second vessel containing a phosphate buffer solution at pH = 4.5, and kept in this second solution under stirring for 1 hour. Finally, the tablet is removed from the second vessel, immersed in a third solution of phosphate buffer at pH = 6.0 contained in a third vessel, and left in this solution under stirring for 6 hours. This protocol simulates the transit of a drug tablet in the Gl tract. Specimens of the three solutions are sampled at given times: a specimen of the first solution is taken after removal of the tablet (total dissolution time at pH = 1.0 of 1 hour); another specimen is taken from the second solution after removal of the tablet (total dissolution time at pH = 4.5 of 1 hour); finally, four specimens are taken from the third solution after 1, 2, 4 and 6 hours of immersion of the tablet in it. All these specimens are subjected to a quantitative HPLC analysis, to measure the amount of clarithromycin dissolved in the different solutions at the given times. The values measured in this test are reported as black squares in the graph of Figure 1, showing the amount of clarithromycin released (as % of the total starting amount in the tablet) as a function of time (h), with the indication of the different pH values at different times.

### EXAMPLE 5 (COMPARATIVE)

The procedure of example 4 is repeated with a tablet of KLACID RM 500 (Abbott Laboratories), containing 500 mg of clarithromycin (namely, the same amount as the tablets of example 4) and being at present the reference solid dosage form for the controlled release of this drug. KLACID RM 500 coated tablets are hydrophilic matrices made of alginates and including anhydrous citric acid. The results of this test are reported as open squares in the graph of Figure 1.

### EXAMPLE 6 (COMPARATIVE)

The test of example 4 is repeated with a tablet produced as described in example 3. The results of this test are reported as open triangles in the graph of Figure 1.

### EXAMPLE 7

The test of example 4 is repeated with a flurithromycin-containing tablet corresponding to Formulation A produced as described in example 2.

In this case, only one specimen of solution at pH = 6.0 is measured, taken after 1 hour of immersion of the tablet in the solution (the tablet is completely dissolved after this period).

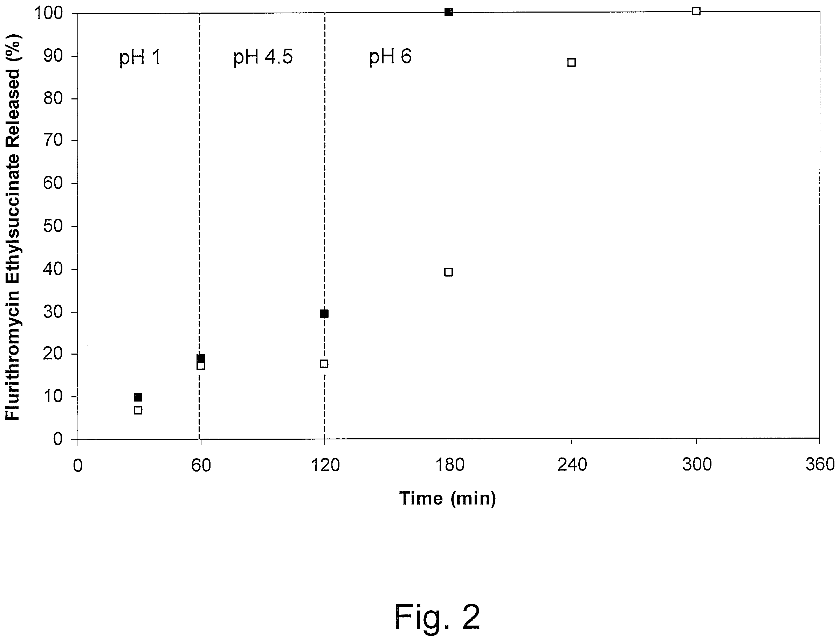

The results of the test are reported in graphic form (black squares) in Figure 2, showing the amount of flurithromycin released (as % of the total starting amount in the tablet) as a function of time (min), with the indication of the different pH values at different times.

### EXAMPLE 8

The test of example 7 is repeated with a flurithromycin-containing tablet corresponding to Formulation B produced as described in example 2.

The results of the test are reported in graphic form (open squares) in Figure 2 as well.

### COMMENTS TO THE RESULTS

The graph in Fig. 1 shows the comparison of prolonged release properties of a drug from a tablet of a composition of the invention prepared according to the second method, compared with a tablet of the reference product, KLACID RM 500 and with a tablet specially produced for comparison purposes; the graph shows that the product of the invention (black squares) is capable of releasing the contained drug (clarithromycin) during a period at least as long as that of the reference (open squares) product (8 hours), and that the rate of release of drug from the product of the invention does not show the decrease of either the reference product or the tablet purposely produced without disgregation promoter (open triangles), indipendently from the pH of the environment, giving rise to a release profile quasi-constant with time. This behavior has to be referred to a shift of delivery mechanism, from diffusive to erosive, of the product of the invention with increasing pH, compared to the delivery mechanism of the reference product and of the tablet without disgregation promoter, that appears to remain diffusive at any pH value.

Fig. 2 shows that tablets containing flurithromycin prepared according to the first preparation method of the invention are also able to release the drug during about 4-5 hours with a delivery profile that does not decrease with increasing pH.

Summarizing these results, it is relevant to observe that the classical behavior of drug delivery with modified release products containing insoluble drugs is characterized by a decrease rate of delivery with pH, such as the case of the reference product or of a formulation similar to that of the invention but without disgregation promoter. The products object of the present invention have the capability to sustain the release rate at nearly neutral pH values until the complete release of drug, giving rise to a kinetics very close to the zero order rate.

Again, this behavior may be referred to the shift of delivery mechanism, from diffusive to erosive, taking place with the dosage forms of the invention with increasing pH. This behavior is confirmed by visual inspection of the dosage forms tested in examples 4, 7 and 8, and of the solutions obtained in the same examples: up to pH 4.5, there is no apparent reduction in size of the dosage forms and the solutions are completely clear and transparent, indicating that the matrix has not undergone any erosion (though the drug is released as evidenced by the graph in Figures 1 and 2); on the other hand, at pH 6.0 there is an evident increase of turbidity of the solutions, and disintegration of the dosage form occurs within few hours, both observations indicating erosion of the matrix into the solution.

## Claims

1. Sustained release composition for poorly soluble drugs having a pH-dependent solubility, in the form of a swellable matrix comprising:
- from about 10 to 30% by weight of a rate controlling hydrophilic swellable polymer being hydroxypropylmethylcellulose having a viscosity value in the range from 4,000 to 15,000 mPa·s., or a blend of two hydroxypropylmethylcellulose fractions of different viscosity, both in the range from 50 to 15,000 mPa·s, the mean viscosity of the blend being comprised between 1,000 and 3,000 mPa·s;
- from about 0.5 to 5% by weight of a disgregation promoter selected among polyethylenglycol of molecular weight between 200 and 8,000 Da; polyethylenoxide of molecular weight lower than about 10,000 Da; polyethylenoxide derivatives obtained by terminating polyethylenoxide chains with either alkyesters with a hydrophilic-lipophilic balance value higher than 15 or sorbitan fatty esters with a hydrophilic-lipophilic balance value higher than 14.9; poloxamers; and polyvinyl alcohol of molecular weight comprised between 40,000 and 100,000 Da;
- from 1 to 5% by weight of polyvinylpyrrolidone having average molecular weight of about 50,000 Da; and
- from 40 to 85% by weight of at least one such drug.

2. Sustained release composition according to claim 1, in which said drug is chosen among neomycin, erythromycin, clarithromycin, flurithromycin, roxithromicin acyclovir and ciprofloxacin, nifedipine, nicardipine.

3. Sustained release composition according to claim 1 in which the disgregation promoter is polyethylenglycol of molecular weight comprised between 1,500 and 8,000 Da.

4. Sustained release composition according to claim 3 in which said polyethylenglycol has a molecular weight of about 6,000 Da.

5. Sustained release composition according to any of claims 1 to 4, further containing one or more excipients and additives chosen among citric acid, mannitol, talc, magnesium stearate, sodium dodecyl sulfate and sodium starch glycolate.

6. Dosage form for sustained release of a poorly soluble drug having a pH-dependent solubility comprising the composition of any of claims 1 to 5.

7. Dosage form according to claim 6, in which said composition is homogeneous throughout the dosage form.

8. Dosage form according to claim 6, made up of granules comprising all the desired components of the composition apart from said hydroxypropylmethylcellulose fraction or fractions, said granules being dispersed in a matrix made up of said hydroxypropylmethylcellulose fraction or fractions.

9. Dosage form according to any of claims 6 to 8, in which the composition is coated with an overlayer.

10. Dosage form according to claim 9, in which the film coating comprises, by weight, about 46.7% of hypromellose, 26.7% of propylene glycol, 2% of sorbitan monooleate, 1.7% vanillin, 2% of hydroxypropyl cellulose, 1.7% of sorbic acid and 19.3% of titanium dioxide.

11. Dosage form according to claim 9, in which the film coating has a thickness comprised between about 100 e 500 µm.

12. Dosage form according to any of claims 6 to 11 in the form of tablets of weight comprised between about 0.6 and 1.2 g, with the higher dimension lower than about 2 cm.

## Patentansprüche

1. Freigabeverzögerungszusammensetzung für schlecht lösliche Arzneimittel mit pH-abhängiger Löslichkeit in der Form einer quellbaren Matrix, umfassend:
- von etwa 10 bis 30 Gewichts-% eines die Rate steuernden hydrophilen quellbaren Polymers, welches Hydroxypropylmethylcellulose mit einem Viskositätswert im Bereich von 4.000 bis 15.000 mPa·s oder eine Mischung aus zwei Hydropropylmethylcellulosefraktionen mit unterschiedlicher Viskosität, beide im Bereich von 50 bis 15.000 mPa·s, ist, wobei die mittlere Viskosität der Mischung zwischen 1.000 und 3.000 mPa·s liegt;
- von etwa 0,5 bis 5 Gewichts-% eines Auflösungsförderers, ausgewählt aus Polyethylenglykol mit einem Molekulargewicht zwischen 200 und 8.000 Da; Polyethylenoxid mit einem Molekulargewicht niedriger als etwa 10.000 Da; Polyethylenoxidderivaten, erhalten durch Abschließen von Polyethylenoxidketten mit entweder Alkylestern mit einem Hydrophil-Lipophil-Gleichgewichtswert höher als 15 oder Sorbitanfettestern mit einem Hydrophil-Lipophil-Gleichgewichtswert höher als 14,9; Poloxameren; und Polyvinylalkohol mit einem Molekulargewicht zwischen 40.000 und 100.000 Da;
- von 1 bis 5 Gewichts-% Polyvinylpyrrolidon mit einem durchschnittlichen Molekulargewicht von etwa 50.000 Da; und
- von 40 bis 85 Gewichts-% zumindest eines solchen Arzneimittels.

2. Freigabeverzögerungszusammensetzung nach Anspruch 1, wobei das Arzneimittel unter Neomycin, Erythromycin, Claritromycin, Flurithromycin, Roxithromicinacyclovir und Ciprofloxacin, Nifedipin, Nicardipin gewählt ist.

3. Freigabeverzögerungszusammensetzung nach Anspruch 1, in welcher der Auflösungsförderer Polyethylenglykol mit einem Molekulargewicht zwischen 1.500 und 8.000 DA ist.

4. Freigabeverzögerungszusammensetzung nach Anspruch 3, in welcher das Polyethylenglykol ein Molekulargewicht von etwa 6.000 Da aufweist.

5. Freigabeverzögerungszusammensetzung nach einem der Ansprüche 1 bis 4, die ferner einen oder mehrere Hilfsstoffe und Additive gewählt aus Zitrinsäure, Mannitol, Talk, Magnesiumstearat, Natriumdodecylsulfat und Natriumstärkeglykolat enthält.

6. Arzneiform zur verzögerten Freigabe eines schlecht löslichen Arzneimittels mit einer pH-abhängigen Löslichkeit, umfassend die Zusammensetzung nach einem der Ansprüche 1 bis 5.

7. Arzneiform nach Anspruch 6, in welcher die Zusammensetzung über die gesamte Arzneiform hinweg homogen ist.

8. Arzneiform nach Anspruch 6, hergestellt aus Körnern, umfassend alle gewünschten Komponenten der Zusammensetzung abgesehen von der/den Hydroxypropylmethylcellulosefraktion oder -fraktionen, wobei die Körner in einer Matrix verteilt sind, die aus der/den Hydroxypropylmethylcellulosefraktion oder -fraktionen hergestellt ist.

9. Arzneiform nach einem der Ansprüche 6 bis 8, in welcher die Zusammensetzung mit einer Deckschicht beschichtet ist.

10. Arzneiform nach Anspruch 9, in welcher die Filmbeschichtung, bezogen auf das Gewicht, etwa 46,7% Hypromellose, 26,7% Propylenglykol, 2% Sorbitanmonooleat, 1,7% Vanillin, 2% Hydroxypropylcellulose, 1,7% Sorbinsäure und 19,3% Titandioxid umfasst.

11. Arzneiform nach Anspruch 9, in welcher die Filmbeschichtung eine Dicke aufweist, die zwischen etwa 100 und 500 µm liegt.

12. Arzneiform nach einem der Ansprüche 6 bis 11 in der Form von Tabletten mit einem Gewicht, das zwischen etwa 0,6 und 1,2 g liegt, wobei die größere Abmessung niedriger als etwa 2 cm ist.

## Revendications

1. Composition à libération prolongée pour des médicaments faiblement solubles ayant une solubilité dépendante du pH, sous forme d'une matrice gonflable, comprenant :
- d'environ 10 à 30 % en poids d'un polymère gonflable hydrophile de régulation de vitesse qui est l'hydroxypropylméthylcellulose ayant une valeur de viscosité dans la plage de 4 000 à 15 000 mPa·s, ou un mélange de deux fractions d'hydroxypropylméthylcellulose de viscosité différente, toutes deux dans la plage de 50 à 15 000 mPa·s, la viscosité moyenne du mélange étant comprise entre 1 000 et 3 000 mPa·s ;
- d'environ 0,5 à 5 % en poids d'un promoteur de désagrégation choisi parmi le poly(éthylène glycol) de poids moléculaire entre 200 et 8 000 Da ; le poly(oxyde d'éthylène) de poids moléculaire inférieur à environ 10 000 Da ; des dérivés de poly(oxyde d'éthylène) obtenus par la terminaison de chaînes de poly(oxyde d'éthylène) soit avec des alkyl esters avec une valeur d'équilibre hydrophile-lipophile supérieure à 15 soit des esters gras de sorbitane avec une valeur d'équilibre hydrophile-lipophile supérieure à 14,9 ; des poloxamères ; et du poly(alcool vinylique) de poids moléculaire compris entre 40 000 et 100 000 Da ;
- de 1 à 5 % en poids de polyvinylpyrrolidone ayant un poids moléculaire moyen d'environ 50 000 Da ; et
- de 40 à 85 % en poids d'au moins un tel médicament.

2. Composition à libération prolongée selon la revendication 1, dans laquelle ledit médicament est choisi parmi la néomycine, l'érythromycine, la clarithromycine, la flurithromycine, la roxithromicine l'acyclovir et la ciprofloxacine, la nifédipine, la nicardipine.

3. Composition à libération prolongée selon la revendication 1, dans laquelle le promoteur de désagrégation est le poly(éthylène glycol) de poids moléculaire compris entre 1 500 et 8 000 Da.

4. Composition à libération prolongée selon la revendication 3, dans laquelle ledit polyéthylène glycol a un poids moléculaire d'environ 6 000 Da.

5. Composition à libération prolongée selon l'une quelconque des revendications 1 à 4, contenant en outre un ou plusieurs excipients et additifs choisis parmi l'acide citrique, le mannitol, le talc, le stéarate de magnésium, le dodécylsulfate de sodium et le glycolate d'amidon de sodium.

6. Forme galénique pour la libération prolongée d'un médicament faiblement soluble ayant une solubilité dépendante du pH comprenant la composition de l'une quelconque des revendications 1 à 5.

7. Forme galénique selon la revendication 6, dans laquelle ladite composition est homogène sur toute la forme galénique.

8. Forme galénique selon la revendication 6, constituée de granules comprenant tous les composants souhaités de la composition à l'exception de ladite ou desdites fraction(s) d'hydroxypropylméthylcellulose, lesdits granules étant dispersés dans une matrice constituée de ladite ou desdites fraction(s) d'hydroxypropylméthylcellulose.

9. Forme galénique selon l'une quelconque des revendications 6 à 8, dans laquelle la composition est enrobée d'une surcouche.

10. Forme galénique selon la revendication 9, dans lequel le pelliculage comprend, en poids, environ 46,7 % d'hypromellose, 26,7 % de propylène glycol, 2 % de monooléate de sorbitane, 1,7 % de vanilline, 2 % d'hydroxypropylcellulose, 1,7 % d'acide sorbique et 19,3 % de dioxyde de titane.

11. Forme galénique selon la revendication 9, dans laquelle le pelliculage a une épaisseur comprise entre environ 100 et 500 µm.

12. Forme galénique selon l'une quelconque des revendications 6 à 11, sous forme de comprimés de poids compris entre environ 0,6 et 1,2 g, la dimension supérieure étant inférieure à environ 2 cm.
